# EUROPÄISCHE PATENTSCHRIFT

(11) **EP 0 443 000 B1**
(45) Veröffentlichungstag und Bekanntmachung des Hinweises auf die Patenterteilung: **25.01.1995**
(21) Anmeldenummer: 90913165.8
(22) Anmeldetag: 06.09.1990
(51) Int. Cl.: C07J 7/00

(54) **VERFAHREN ZUR HERSTELLUNG VON PROGESTERON-DERIVATEN**
PROCESS FOR PRODUCING PROGESTERONE DERIVATIVES
PROCEDE DE PRODUCTION DE DERIVES DE PROGESTERONE

(30) Priorität: 14.09.1989 DE 3931064
(43) Veröffentlichungstag der Anmeldung: 28.08.1991
(73) Patentinhaber: SCHERING AKTIENGESELLSCHAFT, 13342 Berlin (DE)
(72) Erfinder: HÄUSER, Helmut, Dr., 94300 Orizaba, Veracruz (MX); BERNDT, Hans Detlef, Dr., W-1000 Berlin 38 (DE)
(86) Internationale Anmeldenummer: DE9000688
(87) Internationale Veröffentlichungsnummer: WO9104265

(56) Entgegenhaltungen:
- EP-A- 0 153 001
- DE-A- 3 427 486
- FR-A- 2 082 129
- J. Amer. Chem. Soc., Band 81, November 1959 Pietro de Ruggieri et al.: "17-Hydroxypregnanes from Androstane Compounds", siehe Seite 5725 - Seite 5727

## Beschreibung

Die Erfindung betrifft ein Verfahren zur Herstellung von Progesteron-Derivaten der allgemeinen Formel I 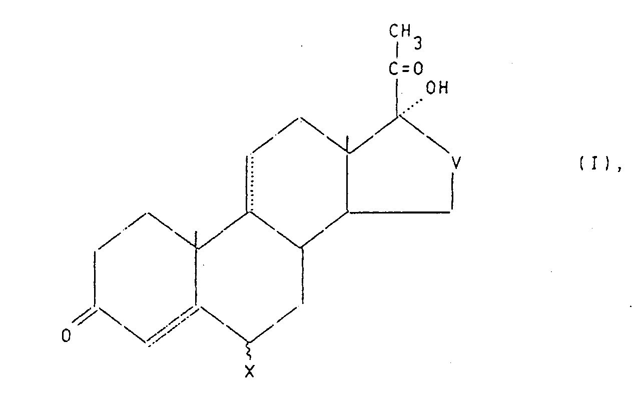
worin
- ....: eine Einfachbindung oder eine Doppelbindung symbolisiert,
- X: ein Wasserstoffatom, ein Fluoratom oder eine Methylgruppe darstellt und
- V: eine Methylengruppe, eine Ethylengruppe, eine Ethylidengruppe oder eine Vinylidengruppe bedeutet,
welches dadurch gekennzeichnet ist, daß man ein Nitril der allgemeinen Formel II 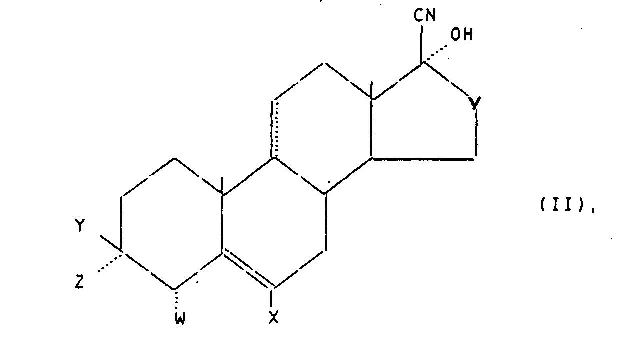
worin
- .....,: X und V die oben genannte Bedeutung besitzen und worin
- Y und Z: gemeinsam eine Alkylendioxygruppe mit 2 bis 6 Kohlenstoffatomen bedeuten und W ein Wasserstoffatom darstellt oder worin
- Y: eine Alkyloxygruppe mit bis zu 4 Kohlenstoffatomen,
eine Benzyloxygruppe oder eine Acyloxygruppe mit 2 bis 8 Kohlenstoffatomen symbolisiert und Z und W zwei Wasserstoffatome darstellen oder gemeinsam eine Kohlenstoff-Kohlenstoff-Bindung bedeuten,
mit einem Grignard-Reagenz der allgemeinen Formel III

CH₃-Mg-Z' (III),

worin Z' ein Chloratom oder ein Bromatom symbolisiert, umsetzt und die gebildeten Zwischenprodukte mittels Säuren hydrolysiert.

Unter einer Alkylendioxygruppe Y und Z der Nitrile der allgemeinen Formel II soll beispielsweise eine 1,2-Ethylendioxygruppe, eine 1,3-Propylendioxygruppe,eine 2,2-Dimethyl-1,3-propylendioxygruppe oder eine 2,3-Butylendioxygruppe verstanden werden.

Geeignete Alkoxygruppen Y sind beispielsweise die Methoxygruppe, die Ethoxygruppe, die Isopropyloxygruppe oder die tert.-Butyloxygruppe.

Geeignete Acyloxygruppen sind unter anderem die Acetoxygruppe, die Propionyloxygruppe, die Dimethylacetoxygruppe, die Trimethylacetoxygruppe und die Benzoyloxygruppe.

Das für die Durchführung des erfindungsgemäßen Verfahrens benötigte Grignard-Reagenz kann in üblicher Weise hergestellt werden, beispielsweise indem man Methylchlorid oder Methylbromid in einem Ether wie Diethylether, Diisopropylether, Dibutylether, 1,2-Dimethoxyethan oder Tetrahydrofuran mit Magnesiumspänen umsetzt. Wegen seines relativ niedrigen Siedepunktes und nicht zu niedrigen Flammenpunktes ist Tetrahydrofuran besonders geeignet. Diese Grignard-Reagenzien können direkt zur Umsetzung mit den Nitrilen der allgemeinen Formel II verwendet werden. Zweckmäßigerweise wird aber vor der Umsetzung der Ether durch Destillation weitgehend entfernt und durch Toluol ersetzt. (Es sei angemerkt, daß Toluol gegenüber Benzol den Vorteil der wesentlich geringeren Toxizität und gegenüber den Xylolen den Vorteil des niedrigen Siedepunktes hat). Dies hat nicht nur den Vorteil daß ein Großteil des Ethers problemlos wiedergewonnen werden kann, sondern darüberhinaus, daß die Reaktion unter milden Bedingungen durchgeführt werden kann und die Aufarbeitung der Reaktionsmischung sehr erleichtert wird.

Zur Durchführung der Reaktion werden zur Erzielung hoher Ausbeuten an Verfahrensprodukt mindestens 4 Mol Grignard-Reagenz benötigt. Die Reaktion wird bei einer Temperatur von -20° C bis +10° C - vorzugsweise bei -5° C bis +5° C - durchgeführt. Unter diesen Bedingungen beträgt die Reaktionszeit etwa 30 bis 180 Minuten.

Nach erfolgter Umsetzung wird die Reaktionsmischung wie bei Grignard-Reaktionen üblich zersetzt. Diese Zersetzung kann beispielsweise mit einer wässrigen Lösung des Tetranatriumsalzes der Ethylendiamintetraessigsäure erfolgen, jedoch ist dieses Reagenz etwas aufwendig. Als recht zweckmäßig und problemlos hat sich die Zersetzung der Reaktionsmischung mit wässriger Ammoniumchlorid-Lösung erwiesen.

Nach erfolgter Zersetzung kann das Toluol mittels Wasserdampfdestillation entfernt werden und man erhält ein Zwischenprodukt, welches aus einem Gemisch des Progesteron-Derivates der allgemeinen Formel I und den Iminoverbindungen der Formeln IV und V 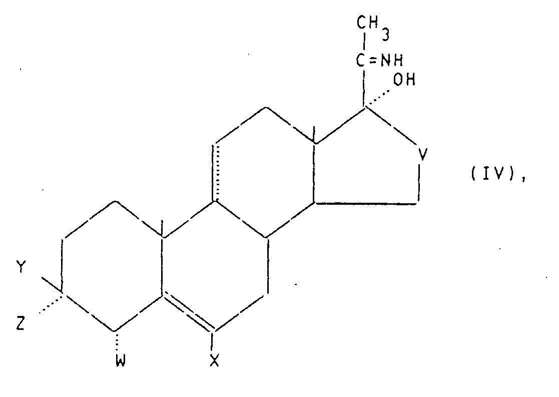 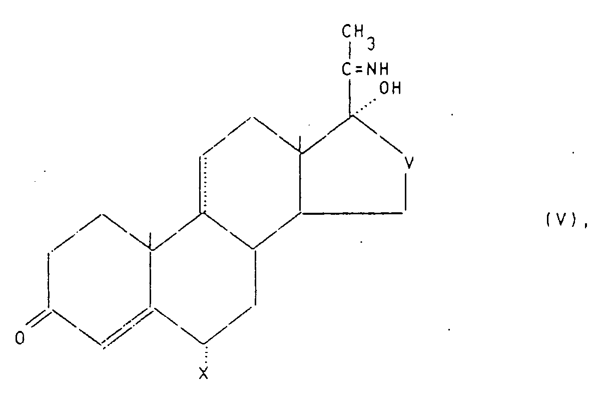
(in diesen Formeln haben ....., X, Y,Z,W und V die oben genannte Bedeutung) besteht.

Dieses Produkt kann problemlos mittels Säuren zu den Progesteron-Derivaten der allgemeinen Formel I hydrolysiert werden.

Diese Hydrolyse läßt sich beispielsweise so durchführen, daß man das Zwischenprodukt in einem niederen Alkohol - wie Methanol, Ethanol oder Isopropanol löst, mit einer Säure, wie Salzsäure, Schwefelsäure oder p-Toluolsulfonsäure versetzt und erhitzt. Nach erfolgter Umsetzung kann das Reaktionsprodukt beispielsweise mit Wasser ausgefällt werden, wobei man zweckmäßigerweise dafür Sorge trägt, daß die Säure neutralisiert wird.

Nach den bislang vorliegenden Versuchen betägt die Ausbeute an Verfahrensprodukt ca. 90 % der Theorie. Sie kann möglicherweise durch Optimierung der Verfahrensparameter weiter gesteigert werden.

Es ist für den Fachmann sehr überraschend, daß die Progesteron-Derivate der allgemeinen Formel I durch direkte Grignardierung von Nitrilen der allgemeinen Formel II hergestellt werden können.

Bereits 1959 hatten P. de Ruggieri et. al. aus Cyanhydrinen von 17-Oxosteroiden 17α-Hydroxy-pregnan-20-on-Derivate hergestellt. Sie hatten zu diesem Zweck die 17-Hydroxygruppe der Cyanhydrine durch Umsetzen mit 2,3-Dihydropyran verethert und dann den 17-Tetrahydropyranylether mit einem Grignard-Reagenz der Formel III umgesetzt. Die direkte Grignardierung von ungeschützten Cyanhydrinen ist nach Meinung der Autoren nicht möglich, da das Grignard-Reagenz zur Dissoziation des Cyanhydrins führt, wobei das Ausgangsketon entsteht, welches mit dem Grignard-Reagenz das entsprechende Methylcarbinol bildet. (J. Amer. Chem. Soc., 81, 1959, p 5725).

Auch L.F. Fieser et. al. (L.F. Fieser und M. Fieser "Steroide" Verlag Chemie, Weinheim DE, 1961, p 742) und E. Oliveto (J. Fried and J.E. Edwards (Ed) "Organic Reactions in Steroid Chemistry" Vol. II; van Nostrand Reinhold Comp. New York et. al. 1972, p 132) vertraten die Ansicht, die direkte Grignardierung von ungeschützten Cyanhydrinen sei nicht möglich.

Wenn Synthesen dieser Art durchgeführt wurden, wurden selbst in neuerer Zeit als Ausgangssubstanzen stets Cyanhydrine verwendet, deren 17-Hydroxygruppe geschützt war. Siehe beispielsweise
K. Annen et. al. DE-A 34 27 486
I. Nitta et al. Bull Chem. Soc., Jpn. 58, 1985, 978
V. H. van Rhennen US-A 4,500,461
J.V.M. Batist et. al. EP-A 0263569 und
A.E.V. Popova et. al. ref. C.A. 102, 1985, 167.005e
Die Vorteile des erfindungsgemäßen Verfahrens liegen nicht nur darin, daß ein Reaktionsschritt eingespart wird, sie liegen auch in der sehr einfachen Durchführbarkeit des erfindungsgemäßen Verfahrens und der hohen Ausbeute an Verfahrensprodukt.

Das nachfolgende Ausführungsbeispiel dient zur näheren Erläuterung des erfindungsgemäßen Verfahrens:

### Ausführungsbeispiel

Aus 219 l einer 3 molaren Lösung von Methylmagnesiumchlorid in Tetrahydrofuran werden unter Stickstoff 100 l Tetrahydrofuran bei Normaldruck abdestilliert. Dann setzt man der Mischung 725 l Toluol so zu, daß die Temperatur 80° C nicht untersteigt und destilliert 300 l Tetrahydrofuran-Toluol Mischung ab.

Man läßt die Lösung auf 0° C erkalten, versetzt sie unter Rühren innerhalb von 30 Minuten mit einer Suspension von 50 kg 3,3-Ethylendioxy-17β-cyano-5-androsten-17α-ol in 150 l Toluol, wobei die Temperatur 5° C nicht übersteigen soll und rührt noch eine Stunde lang bei 5° C.

Dann setzt man der Reaktionsmischung eine Lösung von 25 kg Ammoniumchlorid in 200 l Wasser so zu, daß die Reaktionstemperatur 80° C nicht übersteigt, säuert sie mit Salzsäure an bis zum pH-6,5 erwärmt sie 20 Minuten lang unter Rühren auf 80° C, trennt die organische Phase ab, wäscht die wässrige Phase mit 200 l Toluol, vereinigt die organischen Phasen, versetzt sie mit 200 l Wasser und destilliert das Toluol mittels Wasserdampfdestillation ab.

Nach Erkalten des Reaktionsgemisches wird das abgeschiedene Zwischenprodukt abfiltriert, mit Wasser gewaschen, in 500 l Methanol eingetragen, mit 6,5 l konzentrierter Salzsäure versetzt und 15 Minuten unter Rückfluß erhitzt. Dann läßt man die Mischung erkalten, trägt sie in eine Lösung von 12,5 kg Natriumacetat in 25 l Wasser ein, destilliert 400 l Methanol ab und versetzt den Rückstand langsam mit 500 l Wasser.

Das abgeschiedene Produkt wird abfiltriert, mit Wasser gewaschen und bei 50° C im Umlufttrockenschrank getrocknet.

Man erhält so 48 kg 17α-Hydroxy-4-pregnen-3,20-dion vom Schmelzpunkt 210-213° C. (Reinheit laut HPCL 93 %).

## Patentansprüche

1. Verfahren zur Herstellung von Progesteron-Derivaten der allgemeinen Formel I 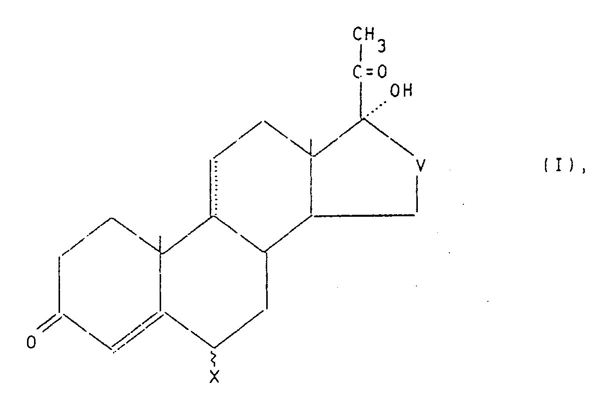 worin
.... eine Einfachbindung oder eine Doppelbindung symbolisiert,
X ein Wasserstoffatom, ein Fluoratom oder eine Methylgruppe darstellt und
V eine Methylengruppe, eine Ethylengruppe, eine Ethylidengruppe oder eine Vinylidengruppe bedeutet,
dadurch gekennzeichnet, daß man ein Nitril der allgemeinen Formel II 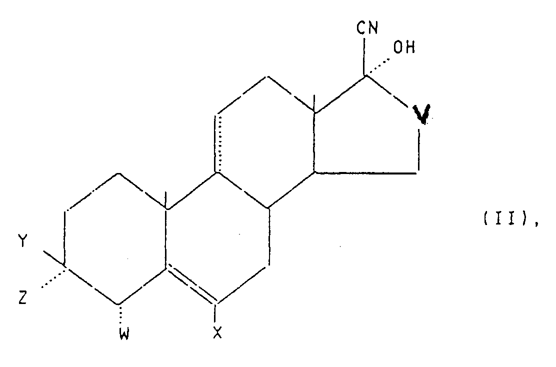 worin
....., X und V die oben genannte Bedeutung besitzen und worin
Y und Z gemeinsam eine Alkylendioxygruppe mit 2 bis 6 Kohlenstoffatomen bedeuten und W ein Wasserstoffatom darstellt oder worin
Y eine Alkyloxygruppe mit bis zu 4 Kohlenstoffatomen,
eine Benzyloxygruppe oder eine Acyloxygruppe mit 2 bis 8 Kohlenstoffatomen symbolisiert und Z und W zwei Wasserstoffatome darstellen oder gemeinsam eine Kohlenstoff-Kohlenstoff-Bindung bedeuten,
mit einem Grignard-Reagenz der allgemeinen Formel III
CH₃-Mg-Z' (III),
worin Z' ein Chloratom oder ein Bromatom symbolisiert, umsetzt und die gebildeten Zwischenprodukte mittels Säuren hydrolysiert.

2. Verfahren zur Herstellung von Progesteron-Derivaten der allgemeinen Formel I gemäß Patentanspurch 1, dadurch gekennzeichnet, daß man die Reaktion mit einer Lösung eines Komplexes des Grignard-Reagenzes mit einem Ether in Toluol durchführt.

3. Verfahren zur Herstellung von Progesteron-Derivaten der allgemeinen Formel I gemäß Patentanspruch 1 und 2, dadurch gekennzeichnet, daß man der Reaktion bei einer Temperatur von -20° C bis +10° C durchführt.

## Claims

1. Process for the manufacture of progesterone derivatives of the general formula I 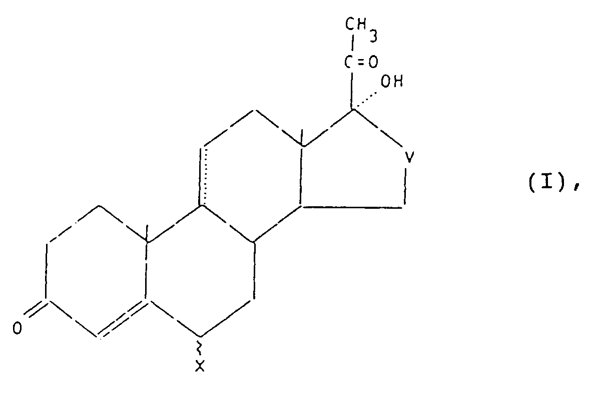 wherein
.... represents a single bond or a double bond,
X represents a hydrogen atom, a fluorine atom or a methyl group and
V represents a methylene group, an ethylene group, an ethylidene group or a vinylidene group,
characterised in that a nitrile of the general formula II 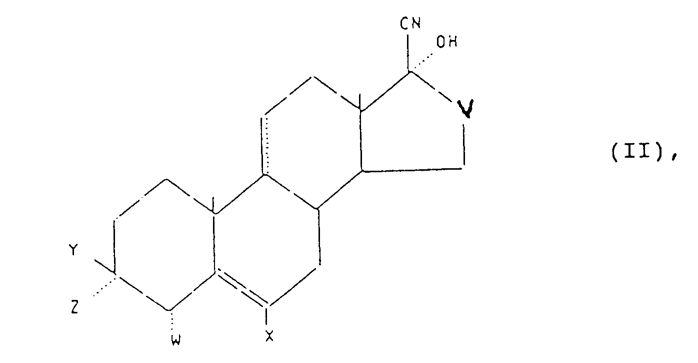 wherein
...., X and V have the meanings mentioned above and
wherein
Y and Z together represent an alkylenedioxy group having from 2 to 6 carbon atoms and
W represents a hydrogen atom or wherein
Y represents an alkyloxy group having up to 4 carbon atoms, a benzyloxy group or an acyloxy group having from 2 to 8 carbon atoms and
Z and W represent two hydrogen atoms or together represent a carbon-carbon bond,
is reacted with a Grignard reagent of the general formula III
CH₃-Mg-Z' (III),
wherein Z' represents a chlorine atom or a bromine atom, and the resulting intermediates are hydrolysed by means of acids.

2. Process for the manufacture of progesterone derivatives of the general formula I according to patent claim 1, characterised in that the reaction is carried out with a solution of a complex of the Grignard reagent with an ether in toluene.

3. Process for the manufacture of progesterone derivatives of the general formula I according to patent claims 1 and 2, characterised in that the reaction is carried out at a temperature of from -20°C to +10°C.

## Revendications

1. Procédé de préparation de dérivés de progestérone de formule générale I ci-dessous : 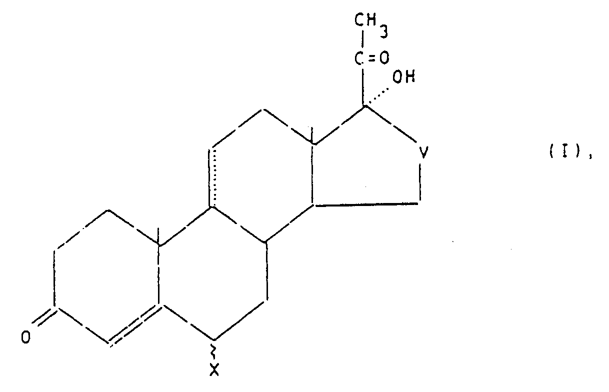 (dans laquelle
.... représente une liaison simple ou double,
X un atome d'hydrogène ou de fluor ou le groupe méthyle et
V un groupe méthylène, éthylène, éthylidène ou vinylidène),
procédé caractérisé en ce que l'on fait réagir un nitrile de formule générale II 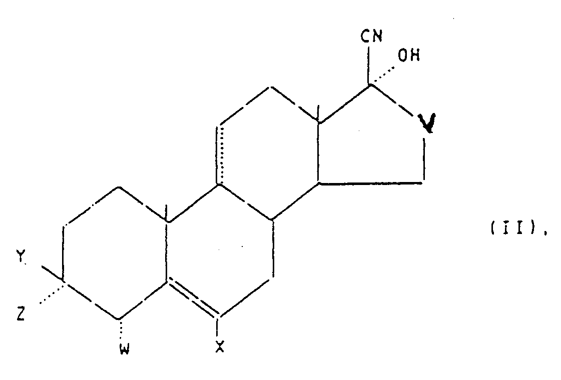 (dans laquelle
les symboles .... X et V ont les mêmes significations que dans la formule I,
Y et Z forment ensemble un groupe alkylène-dioxy ayant de 2 à 6 atomes de carbone et W représente un atome d'hydrogène, ou bien
Y est un alkyloxy pouvant avoir jusqu'à 4 atomes de carbone, un benzyloxy ou un acyloxy ayant de 2 à 8 atomes de carbone, et Z et W sont chacun l'hydrogène ou forment ensemble une liaison carbone-carbone)
avec un réactif de Grignard de formule III
CH₃-Mg-Z' (III),
Z' désignant un atome de chlore ou de brome, puis on hydrolyse avec un acide le produit intermédiaire formé.

2. Procédé selon la revendication 1, caractérisé en ce que l'on effectue la réaction avec une solution d'un complexe du réactif de Grignard et d'un éther dans du toluène.

3. Procédé selon la revendication 1 et/ou 2, caractérisé en ce que l'on effectue la réaction à une température comprise entre -20°C et +10°C.
